# EUROPEAN PATENT APPLICATION

(11) **EP 4 393 468 A1**
(43) Date of publication of application: **03.07.2024**
(21) Application number: 22307052.5
(22) Date of filing: 28.12.2022
(51) Int. Cl.: A61K 6/20, A61K 6/76, C04B 28/18

(54) **DENTAL ANHYDROUS VARNISH COMPOSITION**

(71) Applicant: Septodont ou Septodont SAS ou Specialites Septodont, 94100 Saint Maur des Fossés (FR)
(72) Inventor: RICHARD, Gilles, 91560 CROSNE (FR); BOURGEOIS, Etienne, 93100 MONTREUIL (FR); CALIME, Marie, 75012 PARIS (FR); GUERIN, Julie, 91400 ORSAY (FR); MARIE, Olivier, 91450 SOISY SUR SEINE (FR)
(74) Representative: Icosa

(57) **Abstract**

The present invention relates to the field of varnishes. Especially, the present invention relates to a dental anhydrous varnish composition comprising at least one calcium silicate compound, preferably in an amount ranging from 0.1 % to 20 % by weight relative to the total weight of the composition, at least one film-forming agent, and at least one volatile organic solvent. The present invention further relates to the composition according to the invention for use in the treatment and/or the prevention of hypomineralization related pathologies. Finally, the present invention relates as well to a dental product and to a dental adhesive.

## Description

### FIELD OF INVENTION

The present invention relates to the field of dental varnishes. Especially, the present invention relates to a dental anhydrous varnish composition comprising at least one calcium silicate compound, preferably in an amount ranging from 0.1 % to 20 % by weight relative to the total weight of the composition, at least one film-forming agent, and at least one volatile organic solvent. The present invention further relates to the composition according to the invention for use in the treatment and/or the prevention of hypomineralization related pathologies and/or acid attacks. Finally, the present invention relates as well to a dental product and to a dental adhesive film.

### BACKGROUND OF INVENTION

An increasing number of people have to deal with hypomineralization related pathologies such as white spot lesions (WSL), dental hypersensitivity and molar incisive hypomineralization (MIH). These pathologies are characterized by an alteration of the integrity of tooth hard tissues such as for example of the enamel and/or dentin. The demineralization process responsible for the lesions of tooth hard tissues is characterized by a decrease of the pH saliva (under 5.5) and by acid secretion through the biofilm. This can be the consequence of a poor oral hygiene, of the alimentation or of other dental pathologies. This physiological environment allows the diffusion of H⁺ ions within enamel and dentin. Dental tissues are demineralized by the dissolution of hydroxyapatite minerals into phosphate and calcium ions as expressed in the following:

Ca₁₀(PO₄)₆(OH)₂ + 14H⁺ ↔ 10Ca²⁺ + 6H₃PO₄⁻ + 2H₂.

The remineralization process is induced by a high concentration of calcium and phosphate ions in the saliva. Once saliva is saturated by these ions, the pH will be more alkaline. Consequently, precipitation into calcium carbonate and calcium phosphates occurs on the surface of tooth hard tissues as expressed in the following:

10Ca²⁺ + 6H₃PO₄⁻ + 2H₂O ↔ Ca₁₀(PO₄)₆(OH)₂ + 14H⁺.

In the field of dental products, there is always a need to provide dental compositions with a greater ability for remineralizing tooth hard tissues such as enamel and/or dentin.

Additionally, there is always a need to provide remineralizing dental composition with a good stability overtime and exhibiting excellent occlusion properties, thereby leading to a reduction of pain related to hypersensitivity by tubuli blocage.

Some of known commercial compositions for remineralization of the tooth hard tissues comprise resin substances which sequester part of the active principles and therefore lower the remineralizing effect.

Thus, there is an increasing interest for composition displaying acid penetration prevention properties. Indeed, as indicated before, an anormal acidity can be responsible for the formation of lesions of tooth hard tissues via the demineralization process.

Therefore, there is a real need to develop new dental composition for remineralization of the tooth hard tissues such as enamel and/or dentin, that lowers or avoids the use of resin substances while having an equivalent or even increased remineralizing ability and while displaying additional antacid properties.

These aims are achieved by the present invention. Indeed, the use of calcium silicate compounds offers a fast and deep penetration of a dental anhydrous varnish composition according to the present invention into the damaged tissue thanks to an increased hydrophilicity, a low particle size and adequate flow, rheological properties and affinity. In addition, the use of film-forming agents such as film-forming polymers, is responsible for the varnish galenic form of a composition according to the present invention and favors the interaction between the active remineralizing components with enamel and/or dentin. Finally, the use of volatile organic solvents helps minerals to diffuse through lesions of tooth hard tissues.

### SUMMARY

Thus, one object of the present invention refers to a dental anhydrous varnish composition comprising:
at least one calcium silicate compound, preferably in an amount ranging from 0.1 % to 20 % by weight relative to the total weight of the composition;
at least one film-forming agent; and
at least one volatile organic solvent.

According to one embodiment, the at least one calcium silicate compound is chosen from wollastonite, dicalcium silicate C2S, tricalcium silicate C3S and mixtures thereof; preferably from dicalcium silicate C2S, tricalcium silicate C3S and a mixture thereof; more preferably the at least one calcium silicate compound is dicalcium silicate C2S or tricalcium silicate C3S; and even more preferably the at least one calcium silicate compound is tricalcium silicate C3S.

According to one embodiment, the amount of the at least one calcium silicate compound ranges from 0.1 % to 15 % by weight, preferably from 0.5 % to 15 % by weight, more preferably from 1% to 10% by weight, even more preferably from 1% to 8% by weight, and better still from 2.5% to 7.5% by weight relative to the total weight of the composition.

According to one embodiment, the at least one film-forming agent is selected from resins and/or film-forming polymers; said film-forming polymers preferably being selected from polymethacrylates such as polymethylmethacrylates, polyvinylpyrrolidones, polysaccharides such as cellulose, and mixtures or copolymers thereof; more preferably the at least one film-forming polymer is a polysaccharide selected from cellulose ethers, cellulose esters, and mixtures or copolymers thereof; even more preferably the at least one film-forming polymer is a cellulose ether.

According to one embodiment, the amount of the at least one film-forming agent ranges from 1 % to 40 % by weight, preferably from 5 % to 35 % by weight, more preferably from 8 % to 30 % by weight and better still from 10% to 25% by weight relative to the total weight of the composition.

According to one embodiment, the at least one volatile organic solvent is chosen from volatile alcohols, volatile esters and mixtures thereof; preferably from ethanol, ethyl acetate, isoamyl propionate and mixtures thereof; more preferably from ethanol, ethyl acetate and a mixture thereof; and even more preferably the at least one volatile organic solvent is ethanol.

According to one embodiment, the amount of the at least one volatile organic solvent is greater than or equal to 20 % by weight, preferably ranges from 20 % to 95 % by weight, more preferably from 40 % to 90 % by weight, and better still from 60 % to 80% by weight relative to the total weight of the composition.

According to one embodiment, the composition of the invention further comprises at least one remineralizing agent, different from the at least one calcium silicate compound, preferably chosen from fluoride salts, calcium carbonate, calcium sulfate, hydroxyapatite, a bioglass and mixtures thereof; more preferably chosen from sodium fluoride, tin fluoride, potassium fluoride, cesium fluoride, silver fluoride, calcium carbonate, calcium sulfate, nano hydroxyapatite, a bioglass and mixtures thereof; even more preferably from sodium fluoride, nano hydroxyapatite, a bioglass and mixtures thereof; and better still from nano hydroxyapatite, a bioglass and a mixture thereof.

According to one embodiment, the amount of the at least one remineralizing agent, different from the at least one calcium silicate compound, ranges from 0.1 % to 20 % by weight, preferably from 1 % to 19 % by weight, more preferably from 2 % to 18 % by weight, and even more preferably from 2.5 % to 15 % by weight relative to the total weight of the composition.

According to one embodiment, the composition of the invention further comprises at least two distinct remineralizing agents, different from the at least one calcium silicate compound and different from one another.

According to one embodiment, the composition of the invention further comprises at least one stabilizer, different from the at least one film-forming agent, preferably chosen from glycerol, sorbitol, polyethylene glycols, propylene glycol, microcrystalline cellulose, silica and mixtures thereof; more preferably from polyethylene glycols, propylene glycol and a mixture thereof; and even more preferably the at least one stabilizer is a polyethylene glycol, preferably a polyethylene glycol having a molar mass of lower than or equal to 600 g/mol, and more preferably of lower than or equal to 350 g/mol; the amount of the at least one stabilizer preferably ranges from 0.5 % to 10 % by weight, more preferably from 1 % to 8 % by weight, and even more preferably from 1.5 % to 5 % by weight relative to the total weight of the composition.

Another object of the invention refers to the composition of the invention for use in the treatment and/or the prevention of hypomineralization related pathologies and/or acid attacks.

According to one embodiment, the composition of the invention is applied on at least one surface of at least one tooth of a patient.

Another object of the invention refers to a dental product comprising at least one compartment package containing the dental anhydrous varnish composition of the invention.

Another object of the invention refers to a dental adhesive film directly obtained by a method comprising:
- applying the dental anhydrous varnish composition of the invention on at least one surface of at least one tooth of a patient in need thereof; and
- optionally, drying the composition applied on the surface in step a) to obtain the dental adhesive film on said surface.

### DEFINITIONS

In the present invention, the following terms have the following meanings:
**"Administration"** refers to the application of the dental anhydrous varnish composition of the present invention on at least one surface of a tooth of a patient.
**"Anhydrous composition"** refers to a composition in which the amount of water is lower than or equal to 0.5 % by weight, preferably lower than or equal to 0.4 % by weight, more preferably lower than or equal to 0.2 % by weight, and even more preferably lower than or equal to 0.1 % by weight relative to the total weight of the composition. In one embodiment, **"anhydrous composition"** refers to a composition in which the amount of water is 0 % by weight relative to the total weight of the composition.
**"Calcium silicate compound"** refers to a family of compounds that can be produced by reacting calcium oxide and silica in various ratios. According to one embodiment, the expression "calcium silicate" refers to compounds made of calcium and silicate, preferably selected from wollastonite (of formula CaSiO₃), dicalcium silicate C2S (of formula Ca₂SiO₄), tricalcium silicate C3S (of formula Ca₃SiO₅), or any mixtures thereof.
**"Comprising"** or **"comprise"** is to be construed in an open, inclusive sense.
**"Copolymer"** refers to a polymer derived from at least two different monomeric species. In other words, a "copolymer" refers to a polymer obtained by the repetition of at least two different structural units. Copolymers can be alternating, periodic, statistical, random or block copolymers.
**"Dental hypersensitivity"** refers to the pain arising from exposed dentin to a stimuli that cannot be explained as arising from any other form of dental defect or pathology.
**"Film-forming agent"** refers to a substance able to form a film (*i.e.,* a coat) upon application to a surface. When comprised in a varnish composition, the film-forming agent is responsible for the formation of a film after application to a surface and after drying. According to one embodiment, a film-forming agent may be a film-forming polymer, a resin or any mixture thereof.
**"Film-forming polymer"** refers to any polymer able to form a film (*i.e.,* a coat) upon application to a surface. In the present invention, a film-forming polymer is not a resin.
**"Hypomineralization"** refers to a condition in which the amount of minerals in a tooth hard tissue such enamel and/or dentin, is low enough to lead to the softening and discoloration of said tooth hard tissue. Tooth decay or other damage to the tooth can result from tooth hypomineralization.
**"Patient"** refers to any warm-blooded animal, preferably human, who/which is waiting for, or is receiving medical care or is/will be the object of a medical procedure.
**"Polymer"** refers to any chain or material resulting from the multiple repetition of one or several structural units, said structural units being covalently linked to each other.
**"Prevention"** refers to the avoidance of the targeted pathological condition or disorder in a patient which is not affected by said condition or disorder in the first place. The condition or disorder is successfully "prevented" in a patient if, after being administered the composition according to the present invention, said patient does not develop symptoms associated with the specific disease or condition. The parameters for assessing successful prevention of the disease or conditions are readily measurable by routine procedures familiar to a physician.
**"Remineralizing agent"** refers to a substance able to supply calcium, phosphate and/or fluoride ions to a tooth to promote ion deposition into crystal voids of a demineralized tooth hard tissue such as enamel and/or dentin, in order to produce net mineral gain.
**"Resin"** refers to a solid or highly viscous naturally occurring substance, which can be obtained directly from a plant or which can be synthetically prepared. A resin itself is not a polymer. According to the present invention, a resin is not a film-forming polymer as defined above. According to the present invention, a resin is a film-forming agent but not a film-forming polymer as defined above.
**"Stabilizer"** refers to a substance able to slow down or prevent degradation of a product in which it is comprised.
**"Surface of a tooth"** refers to at least one part of a tooth as for example a specific surface (or side) of the tooth; or may refer to the surface of all the sides of the tooth. According to one embodiment, the surface of a tooth may be selected from one or more of: occlusal, mesial, distal, incisal, facial (labial or buccal) and lingual surface.
**"Treatment"** refers to therapeutic treatment wherein the object is to cure or slow down (lessen) the targeted pathological condition or disorder. A patient is successfully "treated" for the condition or disorder if, after being administered the composition according to the present invention, he shows observable and/or measurable reduction in one or more of the symptoms associated with the specific disease or condition and improvement in quality-of-life issues. The parameters for assessing successful treatment and improvement in the disease or conditions are readily measurable by routine procedures familiar to a physician.
**"Vapor pressure"** refers to the pressure exerted by a vapor in thermodynamic equilibrium with its condensed phases (solid or liquid) at a given temperature in a closed system. Vapor pressure can be measured by measuring the boiling temperature of a purified test substance at different pressures in a Cottrell pump.
**"Varnish"** refers to a liquid that can be applied (for instance, painted or brushed) on a surface and which results, after drying, in a film (*i.e*., a coat) deposited on the surface. According to one embodiment, the deposit of the varnish on a surface leads to a protective layer of said surface, after being dried; preferably a hardened protective layer of said surface.
**"Volatile organic solvent"** refers to an organic solvent which, at a temperature equal to or lower than 50°C, has a vapor pressure lower than 1 bar, preferably lower than or equal to 0.8 bar, and more preferably lower than or equal to 0.6 bar.

Further, in the present invention, when referring to a range, "ranging from X to Y" means that X and Y are included in the range.

### DETAILED DESCRIPTION

### Dental anhydrous varnish composition

This invention relates to a varnish composition, preferably an anhydrous varnish composition, more preferably a dental anhydrous varnish composition.

In one embodiment, the dental anhydrous varnish composition comprises:
- at least one calcium silicate compound, preferably in an amount ranging from 0.1 % to 20 %, more preferably from 0.5% to 20%, by weight relative to the total weight of the composition;
- at least one film-forming agent; and
- at least one volatile organic solvent.

The Applicant has surprisingly evidenced that such a composition displays high remineralization properties of the tooth hard tissues such as enamel and/or dentin, and additional antiacid properties. Furthermore, a composition according to the present invention displays a good stability overtime and also exhibits excellent occlusion properties, thereby leading to a reduction of pain related to hypersensitivity by tubuli blocage.

In one embodiment, the composition according to the present invention is not a paste. In one embodiment, the composition according to the present invention is not a cement paste.

### The at least one calcium silicate compound

Advantageously, the at least one calcium silicate compound may be chosen from:
- pure compounds such as wollastonite (of formula CaSiO₃), dicalcium silicate C2S (of formula Ca₂SiO₄), tricalcium silicate C3S (of formula Ca₃SiO₅) and mixtures thereof; preferably from dicalcium silicate C2S, tricalcium silicate C3S and a mixture thereof; more preferably the at least one calcium silicate compound is dicalcium silicate C2S or tricalcium silicate C3S; and even more preferably the at least one calcium silicate compound is tricalcium silicate C3S; and/or
- calcium silicate-based cements such as for example, Portland cements, mineral trioxide aggregates (MTA) and mixtures thereof.

The amount of the at least one calcium silicate compound advantageously ranges from 0.1 % to 15 % by weight, preferably from 0.5 % to 15 % by weight, and more preferably from 1% to 10% by weight, even more preferably from 1% to 8% by weight, and better still from 2.5 % to 7.5 % by weight relative to the total weight of the composition.

In one embodiment, the amount of the at least one calcium silicate compound is 5% by weight relative to the total weight of the composition.

In one embodiment, the at least one calcium silicate compound is dicalcium silicate (C2S) and/or tricalcium silicate (C3S) and its amount advantageously ranges from 0.1% to 15% by weight, preferably from 0.5% to 15% by weight, more preferably from 1% to 10% by weight, even more preferably from 1% to 8% by weight, and better still from 2.5% to 7.5% by weight relative to the total weight of the composition.

In a preferred embodiment, the at least one calcium silicate compound is tricalcium silicate C3S and its amount advantageously ranges from 0.1 % to 15% by weight, preferably from 0.5% to 15% by weight, more preferably from 1% to 10% by weight, even more preferably from 1 % to 8 % by weight, and better still from 2.5 % to 7.5 % by weight relative to the total weight of the composition.

### The at least one film-forming agent

Advantageously, the at least one film-forming agent may be a film-forming polymer, a resin or any mixtures thereof.

In one embodiment, the at least one film-forming polymer is selected from polymethacrylates such as polymethylmethacrylates, polyvinylpyrrolidones, polysaccharides such as cellulose, and mixtures or copolymers thereof; preferably the at least one film-forming polymer is a polysaccharide selected from cellulose ethers, cellulose esters, and mixtures or copolymers thereof; more preferably the at least one film-forming polymer is a cellulose ether; and even more preferably the at least one film-forming polymer is ethylcellulose and/or hydroxypropylcellulose.

In one embodiment, the resin may be selected from any solid or highly viscous naturally occurring substance, which can be obtained directly from a plant or which can be synthetically prepared; preferably the resin is selected from one or more of: amber, kauri gum, dammar, copal, mastic, rosin (colophonium, for instance), sandarac, balsam, elemi and shellac.

In another embodiment of the invention, the composition is free of resin. In other words, in this embodiment, the amount of resin in the composition is lower than or equal to 0.5 % by weight, preferably lower than or equal to 0.4 % by weight, more preferably lower than or equal to 0.2 % by weight, and even more preferably lower than or equal to 0.1 % by weight relative to the total weight of the composition. In an even more preferred embodiment, the amount of resin in the composition is 0 % by weight relative to the total weight of the composition. In one embodiment, when the amount of resin in the composition is 0 % by weight relative to the total weight of the composition, the film-forming agent is a film-forming polymer as defined above.

Advantageously, the amount of the at least one film-forming agent ranges from 1 % to 40 % by weight, preferably from 5 % to 35 % by weight, more preferably from 8 % to 30 % by weight, and even more preferably from 10 % to 20 % by weight relative to the total weight of the composition. In one embodiment, the amount of the at least one film-forming agent is 15% or 20% by weight to the total weight of the composition.

The weight ratio (Ra) between the amount of the at least one film-forming agent and the amount of the at least one calcium silicate compound is advantageously greater than or equal to 1, preferably ranges from 1.2 to 10, more preferably from 1.5 to 8, and even more preferably from 2 to 6.

In one embodiment, the at least one film-forming polymer is a cellulose ether and its amount advantageously ranges from 1 to 40 % by weight, preferably from 5 % to 35 % by weight, more preferably from 8 % to 30 % by weight, and even more preferably from 10 % to 20 % by weight relative to the total weight of the composition.

In a preferred embodiment, the at least one film-forming polymer is ethylcellulose and/or hydroxypropylcellulose and its amount advantageously ranges from 1 to 40 % by weight, preferably from 5 % to 35 % by weight, more preferably from 8 % to 30 % by weight, and even more preferably from 10 % to 20 % by weight relative to the total weight of the composition. In a preferred embodiment, the at least one film-forming polymer is ethylcellulose and/or hydroxypropylcellulose and its amount is 15% or 20%. In a preferred embodiment, the composition of the invention comprises 15% of ethylcellulose and 5% of hydroxypropylcellulose, by weight relative to the total weight of the composition. In a preferred embodiment, the composition of the invention comprises 11.3% of ethylcellulose and 3.7% of hydroxypropylcellulose, by weight relative to the total weight of the composition.

### The at least one volatile organic solvent

Advantageously, the at least one volatile organic solvent is chosen from volatile alcohols, volatile esters and mixtures thereof; preferably from ethanol, ethyl acetate, isoamyl propionate and mixtures thereof; more preferably from ethanol, ethyl acetate and a mixture thereof; and even more preferably the at least one volatile organic solvent is ethanol.

In one embodiment, ethanol is absolute ethanol.

Advantageously, the amount of the at least one volatile organic solvent is greater than or equal to 20 % by weight, preferably ranges from 20 % to 95 % by weight, more preferably from 40 % to 90 % by weight, even more preferably from 60 % to 80 %, and better still from 65% to 75% by weight relative to the total weight of the composition.

The weight ratio (Rb) between the amount of the at least one volatile organic solvent and the amount of the at least one calcium silicate compound is advantageously greater than or equal to 5, preferably ranges from 6 to 30, more preferably from 9 to 26, and even more preferably from 10 to 24.

In one embodiment, the at least one volatile organic solvent is chosen from ethanol, ethyl acetate and a mixture thereof and its amount is advantageously greater than or equal to 20 % by weight, preferably ranges from 20 % to 95 % by weight, more preferably from 40 % to 90 % by weight, and even more preferably from 60 % to 80 %, and better still from 65% to 75% by weight relative to the total weight of the composition.

In a preferred embodiment, the at least one volatile organic solvent is ethanol, preferably absolute ethanol, and its amount is advantageously greater than or equal to 20 % by weight, preferably ranges from 20 % to 95 % by weight, more preferably from 40 % to 90 % by weight, and even more preferably from 60 % to 80 %, and better still from 65% to 75%, by weight relative to the total weight of the composition.

In a preferred embodiment, the at least one volatile organic solvent is ethanol, preferably absolute ethanol, and its amount is 37.5%, 65%, 70%, 73%, 74.5%, or 75%, by weight relative to the total weight of the composition.

In a preferred embodiment, the at least one volatile organic solvent is ethyl acetate and its amount is 37.5% or 75%, by weight relative to the total weight of the composition.

In a preferred embodiment, the volatile organic solvent is a mixture of ethyl acetate and ethanol, and the amount of ethyl acetate is 37.5% and the amount of ethanol is 37.5%, by weight relative to the total weight of the composition.

### Other compounds

The dental anhydrous varnish composition according to the present invention may optionally further comprise at least one remineralizing agent, different from the at least one calcium silicate compound. The addition of said at least one remineralizing agent, different from the at least one calcium silicate compound, allows for a better remineralization.

The at least one remineralizing agent, different from the at least one calcium silicate compound, when present in the composition according to the present invention, is preferably chosen from fluoride salts, calcium carbonate, calcium sulfate, hydroxyapatite, a bioglass and mixtures thereof; more preferably from sodium fluoride, tin fluoride, potassium fluoride, cesium fluoride, silver fluoride, calcium carbonate, calcium sulfate, nano hydroxyapatite, a bioglass and mixtures thereof; even more preferably from sodium fluoride, nano hydroxyapatite, a bioglass and mixtures thereof; and better still from nano hydroxyapatite, a bioglass and a mixture thereof. Examples of bioglass remineralizing agents are bioglass 58S and bioglass 45S.

Advantageously, the amount of the at least one remineralizing agent, different from the at least one calcium silicate compound, when present in the composition according to the present invention, ranges from 0.5 % to 20 % by weight, preferably from 1 % to 19 % by weight, more preferably from 2 % to 18 % by weight, and even more preferably from 2.5 % to 15 % by weight relative to the total weight of the composition.

In one embodiment, the amount of the at least one remineralizing agent, different from the at least one calcium silicate compound, when present in the composition according to the present invention, is 5% or 10% by weight relative to the total weight of the composition.

When present in the composition according to the present invention, the weight ratio (Rc) between the amount of the at least one remineralizing agent different from the at least one calcium silicate compound, and the amount of the at least one calcium silicate compound is advantageously greater than or equal to 1, preferably ranges from 1 to 3, and more preferably from 1 to 2.

In one embodiment, the composition according to the present invention may further comprise at least one remineralizing agent, different from the at least one calcium silicate compound, chosen from sodium fluoride, nano hydroxyapatite, a bioglass and mixtures thereof; and the amount of said at least one remineralizing agent advantageously ranges from 0.5 % to 20 % by weight, preferably from 1 % to 19 % by weight, more preferably from 2 % to 18 % by weight, and even more preferably from 2.5 % to 15 %, and better still is 5% or 10%, by weight relative to the total weight of the composition.

In a preferred embodiment, the composition according to the present invention may further comprise at least one remineralizing agent, different from the at least one calcium silicate compound, chosen from nano hydroxyapatite, a bioglass and a mixture thereof and its amount advantageously ranges from 0.5 % to 20 % by weight, preferably from 1 % to 19 % by weight, more preferably from 2 % to 18 % by weight, and even more preferably from 2.5 % to 15 % by weight, and better still is 5% or 10% by weight relative to the total weight of the composition.

In one embodiment, the composition according to the present invention may further comprise at least two distinct remineralizing agents different from the at least one calcium silicate compound and different from one another. Advantageously, in this embodiment, the at least two distinct remineralizing agents are chosen from nano hydroxyapatite, bioglasses and mixtures thereof, and the total amount of said at least two distinct remineralizing agents, different from the at least one calcium silicate compound and different from one another, advantageously ranges from 0.5 % to 20 % by weight, preferably from 1 % to 19 % by weight, more preferably from 2 % to 18 % by weight, and even more preferably from 2.5 % to 15 % by weight relative to the total weight of the composition. In a preferred variant of this embodiment, the composition according to the present invention further comprises a mixture of nano hydroxyapatite and a bioglass, in an amount that advantageously ranges from 0.5 % to 20 % by weight, preferably from 1 % to 19 % by weight, more preferably from 2 % to 18 % by weight, and even more preferably from 2.5 % to 15 % by weight relative to the total weight of the composition. The composition according to this embodiment displays even better occlusion properties.

The composition according to the present invention may optionally further comprise at least one stabilizer, different from the film-forming agent as defined above.

The at least one stabilizer, when present in the composition according to the present invention, is preferably chosen from glycerol, sorbitol, polyethylene glycols, propylene glycol, microcrystalline cellulose, silica such as for example fumed silica, and mixtures thereof; more preferably from polyethylene glycols, propylene glycol and a mixture thereof; and even more preferably the at least one stabilizer is a polyethylene glycol, preferably a polyethylene glycol having a molar mass of lower than or equal to 600 g/mol, more preferably lower than 400 g/mol, and even more preferably lower than or equal to 350 g/mol. Examples of polyethylene glycols suitable in the composition of the present invention are PEG 200, PEG 300 and PEG 600.

The amount of the at least one stabilizer, when present in the composition according to the present invention, preferably ranges from 0.5 % to 10 % by weight, more preferably from 1 % to 8 % by weight, and even more preferably from 1.5 % to 5 % by weight, and better still is 2%, by weight relative to the total weight of the composition.

When present in the composition according to the present invention, the weight ratio (Rd) between the amount of the at least one stabilizer, and the amount of the at least one calcium silicate compound is advantageously greater than or equal to 0.4, preferably ranges from 0.5 to 1.5, and more preferably from 0.5 to 1.33.

In one embodiment, the composition according to the present invention further comprises at least one stabilizer chosen from polyethylene glycols, propylene glycol and a mixture thereof and its amount advantageously ranges from 0.5 % to 10 % by weight, preferably from 1 % to 8 % by weight, and more preferably from 1.5 % to 5 % by weight, and better still is 2%, by weight relative to the total weight of the composition.

In a preferred embodiment, the composition according to the present invention further comprises a polyethylene glycol and its amount advantageously ranges from 0.5 % to 10 % by weight, preferably from 1 % to 8 % by weight, and more preferably from 1.5 % to 5 % by weight, and better still is 2%, by weight relative to the total weight of the composition.

In another embodiment, the composition according to the present invention further comprises one remineralizing agent, different from the at least one calcium silicate compound, and one stabilizer. Advantageously, in this embodiment, the remineralizing agent is preferably chosen from sodium fluoride, tin fluoride, potassium fluoride, cesium fluoride, silver fluoride, calcium carbonate, calcium sulfate, nano hydroxyapatite, and a bioglass; more preferably from sodium fluoride, nano hydroxyapatite, and a bioglass; and even more preferably the remineralizing agent is nano hydroxyapatite; the amount of said remineralizing agent advantageously ranges from 0.5 % to 20 % by weight, preferably from 1 % to 19 % by weight, more preferably from 2 % to 18 % by weight, and even more preferably from 2.5 % to 15 % by weight relative to the total weight of the composition; the stabilizer is preferably chosen from glycerol, sorbitol, polyethylene glycols, and propylene glycol; more preferably from polyethylene glycols and propylene glycol; and even more preferably the stabilizer is a polyethylene glycol; the amount of said stabilizer advantageously ranges from 0.5 % to 10 % by weight, preferably from 1 % to 8 % by weight, and more preferably from 1.5 % to 5 % by weight relative to the total weight of the composition. The composition according to this embodiment also displays even better occlusion properties.

The composition according to the present invention may optionally further comprise one or more additional compounds, different from the compounds defined above and preferably chosen from masking agents, antibacterial agents, fragrances, flavors such as for example xylitol, pigments, colorants, other texturing agents and mixtures thereof.

Preferably, when the additional compounds above are present in the composition according to the invention, the additional compounds are present in general in an amount each of between 0.01 % and 20 % by weight relative to the weight of the composition. Needless to say, a person skilled in the art will take care to select these optional additional compounds such that the advantageous properties intrinsically associated with the composition of the invention are not, or are not substantially, adversely affected by the envisaged addition(s).

### Preferred dental anhydrous varnish compositions

According to one embodiment, the composition according to the present invention comprises:
- dicalcium silicate C2S or tricalcium silicate C3S, which amount advantageously ranges from 0.1 % to 20 % by weight, preferably from 0.1 % to 15 % by weight, preferably from 0.5 % to 15 % by weight, more preferably from 1% to 10% by weight, even more preferably from 1% to 8% by weight, and better still from 2.5% to 7.5% by weight relative to the total weight of the composition;
- a cellulose ether, which amount advantageously ranges from 1 to 40 % by weight, preferably from 5 % to 35 % by weight, more preferably from 8 % to 30 % by weight, and even more preferably from 10 % to 20 % by weight relative to the total weight of the composition;
- at least one volatile organic solvent chosen from ethanol, ethyl acetate and a mixture thereof; the amount of said at least one volatile organic solvent is advantageously greater than or equal to 20 % by weight, preferably ranges from 20 % to 95 % by weight, more preferably from 40 % to 90 % by weight, and even more preferably from 60 % to 80 % by weight relative to the total weight of the composition;
- optionally, at least one remineralizing agent, different from dicalcium silicate C2S or tricalcium silicate C3S, which is chosen from sodium fluoride, nano hydroxyapatite, a bioglass and mixtures thereof; the amount of said at least one remineralizing agent advantageously ranges from 0.1 % to 20 % by weight, preferably from 1 % to 19 % by weight, more preferably from 2 % to 18 % by weight, and even more preferably from 2.5 % to 15 % by weight relative to the total weight of the composition; and/or
- optionally, at least one stabilizer chosen from polyethylene glycols, propylene glycol and a mixture thereof; the amount of said at least one stabilizer preferably ranges from 0.5 % to 10 % by weight, more preferably from 1 % to 8 % by weight, and even more preferably from 1.5 % to 5 % by weight relative to the total weight of the composition.

According to a preferred embodiment, the composition according to the present invention comprises:
- tricalcium silicate C3S, which amount advantageously ranges from 0.1 % to 20 % by weight, preferably from 0.1 % to 15 % by weight, preferably from 0.5 % to 15 % by weight, more preferably from 1% to 10% by weight, even more preferably from 1% to 8% by weight, and better still from 2.5% to 7.5% by weight relative to the total weight of the composition;
- ethylcellulose and/or hydroxypropylcellulose, which amount advantageously ranges from 1 to 40 % by weight, preferably from 5 % to 35 % by weight, more preferably from 8 % to 30 % by weight, and even more preferably from 10 % to 20 % by weight relative to the total weight of the composition;
- ethanol, which amount is advantageously greater than or equal to 20 % by weight, preferably ranges from 20 % to 95 % by weight, more preferably from 40 % to 90 % by weight, and even more preferably from 60 % to 80 % by weight relative to the total weight of the composition;
- optionally, at least one remineralizing agent, different from tricalcium silicate C3S, which is chosen from nano hydroxyapatite, a bioglass and a mixture thereof; the amount of said at least one remineralizing agent advantageously ranges from 0.1 % to 20 % by weight, preferably from 1 % to 19 % by weight, more preferably from 2 % to 18 % by weight, and even more preferably from 2.5 % to 15 % by weight relative to the total weight of the composition; and/or
- optionally, a polyethylene glycol, which amount preferably ranges from 0.5 % to 10 % by weight, more preferably from 1 % to 8 % by weight, and even more preferably from 1.5 % to 5 % by weight relative to the total weight of the composition.

According to a first even preferred embodiment, the composition according to the present invention comprises:
- tricalcium silicate C3S, which amount advantageously ranges from 0.1 % to 20 % by weight, preferably from 1 % to 15% by weight, more preferably from 2 % to 9 % by weight, and even more preferably from 2.5 % to 7.5 % by weight relative to the total weight of the composition;
- ethylcellulose and/or hydroxypropylcellulose, which amount advantageously ranges from 1 to 40 % by weight, preferably from 5 % to 35 % by weight, more preferably from 8 % to 30 % by weight, and even more preferably from 10 % to 20 % by weight relative to the total weight of the composition;
- ethanol, which amount is advantageously greater than or equal to 20 % by weight, preferably ranges from 20 % to 95 % by weight, more preferably from 40 % to 90 % by weight, and even more preferably from 60 % to 80 % by weight relative to the total weight of the composition; and
- a mixture of nano hydroxyapatite and a bioglass, the amount of said mixture advantageously ranges from 0.5 % to 20 % by weight, preferably from 1 % to 19 % by weight, more preferably from 2 % to 18 % by weight, and even more preferably from 2.5 % to 15 % by weight relative to the total weight of the composition.

The composition according to this first even preferred embodiment displays even better occlusion properties.

According to a second even preferred embodiment, the composition according to the present invention comprises:
- Tricalcium silicate C3S, which amount advantageously ranges from 0.1 % to 20 % by weight, preferably from 1 % to 15% by weight, more preferably from 2 % to 9 % by weight, and even more preferably from 2.5 % to 7.5 % by weight relative to the total weight of the composition;
- ethylcellulose or hydroxypropylcellulose, which amount advantageously ranges from 1 to 40 % by weight, preferably from 5 % to 35 % by weight, more preferably from 8 % to 30 % by weight, and even more preferably from 10 % to 20 % by weight relative to the total weight of the composition;
- ethanol, which amount is advantageously greater than or equal to 20 % by weight, preferably ranges from 20 % to 95 % by weight, more preferably from 40 % to 90 % by weight, and even more preferably from 60 % to 80 % by weight relative to the total weight of the composition;
- nano hydroxyapatite, which amount advantageously ranges from 0.5 to 20 % by weight, preferably from 1 % to 19 % by weight, more preferably from 2 % to 18 % by weight, and even more preferably from 2.5 % to 15 % by weight relative to the total weight of the composition; and
- a polyethylene glycol, which amount preferably ranges from 0.5 % to 10 % by weight, more preferably from 1 % to 8 % by weight, and even more preferably from 1.5 % to 5 % by weight relative to the total weight of the composition.

The composition according to this second even preferred embodiment displays also even better occlusion properties.

### Use

The invention also relates to the dental anhydrous varnish composition according to the present invention for use thereof in the treatment and/or the prevention of hypomineralization related pathologies, such as white spot lesions (WSL), dental hypersensitivity, molar incisive hypomineralization (MIH) and hypomineralized second primary molars (HSPM).

The invention further relates to the composition according to the present invention for use thereof in the treatment and/or the prevention of white spot lesions (WSL).

The invention relates as well to the composition according to the present invention for use thereof in the treatment and/or the prevention of dental hypersensitivity. In other words, the invention relates to the composition according to the present invention for use thereof in the treatment and/or the prevention of the pain arising from exposed dentin to a stimuli.

In addition, the invention relates to the composition according to the present invention for use thereof in the treatment and/or the prevention of molar incisive hypomineralization (MIH).

The invention also relates to the composition according to the present invention for use thereof in the prevention of caries.

In one embodiment, the composition for use according to the present invention is applied on at least one surface of at least one tooth of a patient, preferably at least two teeth, preferably selected from one or more of: occlusal, mesial, distal, incisal, facial (labial or buccal) and lingual surface; and is preferably painted or brushed.

In one embodiment, the composition for use according to the present invention is applied on all the surfaces of at least one tooth of a patient, preferably selected from one or more of: occlusal, mesial, distal, incisal, facial (labial or buccal) and lingual surface; and is preferably painted or brushed.

### Method for manufacturing the dental anhydrous varnish composition according to the present invention

The invention further relates to a method for manufacturing the dental anhydrous varnish composition according to the present invention, said method comprises the step of mixing (or "mixing step (i)") the at least one calcium silicate compound, the at least one film-forming agent, the at least one volatile organic solvent and optionally, the additional compounds, when they are present in said composition.

In one embodiment, the mixing step (i) of the method of the invention comprises a first step (step (i-1)) for mixing the at least one film-forming agent and the at least one volatile organic solvent; and a second step (step (i-2)) for mixing the resulting mixture of step (i-1) and the at least one calcium silicate compound. In one embodiment, the mixing step (i) of the method of the invention further comprises a third step (step (i-3)) for mixing the resulting mixture of step (i-2) with at least one additional compound as defined above.

Advantageously, steps (i-1), (i-2) and/or optional (i-3) of the method for manufacturing the composition according to the present invention is(are) implemented to a temperature ranging from more than 0°C to 50°C, preferably from 10°C to 40°C, more preferably at about 25°C.

Advantageously, steps (i-1), (i-2) and/or optional (i-3) of the method for manufacturing the composition according to the present invention is(are) implemented under agitation, preferably ranging from 10 rpm to 1000 rpm, more preferably from 100 rpm to 800 rpm, even more preferably from 400 rpm to 600 rpm.

### Dental product

The invention further relates to a dental product comprising at least one compartment package containing the dental anhydrous varnish composition according to the present invention.

Advantageously, the dental product comprises a single compartment package containing the composition according to the present invention. Said single compartment package containing the composition according to the present invention can be a dental applicator, a box, a blister, a vial, a tube, a bottle, a sachet, an ampoule, a flask, a syringe barrel, a pen reservoir, or any single compartment package commonly known by a skilled in the art.

In one embodiment, the dental product according to the present invention is hermetic. In other words, said dental product is both airtight and/or waterproof.

In one embodiment, the dental product according to the present invention may further comprise a brush. In one embodiment, the dental product is a dental applicator brush. The brush can be employed to apply the dental anhydrous varnish composition according to the present invention on at least one surface of a tooth of a patient in need thereof.

### Dental adhesive film

The invention also relates to a dental adhesive film, *i.e.,* a dental adhesive coat, directly obtained by a method comprising the following steps:
a) applying the dental anhydrous varnish composition according to the present invention on at least one surface of at least one tooth of a patient in need thereof; and
b) drying the composition applied on the surface in step a) to obtain the dental adhesive film on said surface.

The presence of the at least one film-forming agent in the composition according to the present invention allows to obtain the dental adhesive film on the at least one surface of a tooth of a patient in need thereof after drying (*i.e.,* after step b)).

Steps a) to b) of the method employed to obtain the dental adhesive film according to the present invention are performed in that order.

In one embodiment, step (a) and/or step (b) of the method may be implemented on at least one surface of at least two teeth of the patient in need thereof; said surface being selected from one or more of: occlusal, mesial, distal, incisal, facial (labial or buccal) and lingual surface.

In one embodiment, the drying of the composition applied in step a) in the method employed to obtain the dental adhesive film according to the present invention occurs via the evaporation of the at least one volatile organic solvent, advantageously at a temperature ranging from 10°C to 40°C, preferably ranging from 20°C and 40°C, even more preferably at about 25°C or at about 37°C.

In one embodiment, the drying of the composition applied in step a) in the method employed to obtain the dental adhesive film according to the present invention occurs via the use of an apparatus. In this case, all the drying methods or apparatus well-known by the skilled artisan can be applied for the drying step (b) of the method of the invention. For example, a dental air dryer may be used for the drying step (b).

### EXAMPLES

The present invention is further illustrated by the following examples.

### Example 1: Examples of dental anhydrous varnish compositions of the present invention

Several compositions according to the present invention have been prepared using the components and amounts indicated in Tables 1-2:

**Table 1**

| N° | | 1 | 2 | 3 | 4 | 5 | 6 | 7 | 8* | 9 |
|---|---|---|---|---|---|---|---|---|---|---|
| Calcium silicate compound (% by weight) | dicalcium silicate C2S | - | 2.5 | - | - | - | - | - | - | - |
| | tricalcium silicate C3S | 5 | 2.5 | 5 | 5 | 5 | 5 | 5 | 5 | 5 |
| Film-forming agent (% by weight) | ethylcellulose | - | - | - | - | - | 15 | - | 15 | 11.3 |
| | hydroxypropylcellulose | 20 | 20 | 20 | 20 | 20 | 5 | - | 5 | 3.7 |
| | polyvidone | - | - | - | - | - | - | 20 | - | - |
| Volatile organic solvent (% by weight) | ethanol | 75 | 75 | 70 | 70 | 65 | 75 | 65 | 74.5 | 75 |
| | ethyl acetate | - | - | - | - | - | - | - | - | - |
| Additional remineralizing agent (% by weight) | sodium fluoride | - | - | - | 5 | 5 | - | 5 | - | - |
| | nano hydroxyapatite | - | - | 5 | - | 5 | - | 5 | - | 5 |
| | tin fluoride | - | - | - | - | - | - | - | - | - |
| | bioglass 58S | - | - | - | - | - | - | - | - | - |
| | bioglass 45S | - | - | - | - | - | - | - | - | - |
| Stabilizer (% by weight) | PEG 200 | - | - | - | - | - | - | - | - | - |
| | PEG 300 | - | - | - | - | - | - | - | - | - |
| | PEG 400 | - | - | - | - | - | - | - | - | - |

**Table 2**

| N° | | 10 | 11 | 12 | 13 | 14 | 15 | 16 | 17 |
|---|---|---|---|---|---|---|---|---|---|
| Calcium silicate compound (% by weight) | dicalcium silicate C2S | - | - | - | - | - | - | - | - |
| | tricalcium silicate C3S | 5 | 5 | 5 | 5 | 5 | 5 | 5 | 5 |
| Film-forming agent (% by weight) | ethylcellulose | 11.3 | 11.3 | 11.3 | 11.3 | 11.3 | 11.3 | 11.3 | 11.3 |
| | hydroxypropylcellulose | 3.7 | 3.7 | 3.7 | 3.7 | 3.7 | 3.7 | 3.7 | 3.7 |
| | polyvidone | - | - | - | - | - | - | - | - |
| Volatile organic solvent (% by weight) | ethanol | - | 37.5 | 70 | 70 | 70 | 73 | 73 | 73 |
| | ethyl acetate | 75 | 37.5 | - | - | - | - | - | - |
| Additional remineralizing agent (% by weight) | sodium fluoride | - | - | - | - | - | - | - | - |
| | nano hydroxyapatite | 5 | 5 | 5 | 5 | 5 | 5 | 5 | 5 |
| | tin fluoride | - | - | 5 | - | - | - | - | - |
| | bioglass 58S | - | - | - | 5 | - | - | - | - |
| | bioglass 45S | - | - | - | - | 5 | - | - | - |
| Stabilizer (% by weight) | PEG 200 | - | - | - | - | - | 2 | - | - |
| | PEG 300 | - | - | - | - | - | - | 2 | - |
| | PEG 400 | - | - | - | - | - | - | - | 2 |

| | | | | | | | | | |
|---|---|---|---|---|---|---|---|---|---|
| *Composition 8 further comprises 0.5% of flavor mint by weight to the total weight of said composition. | | | | | | | | | |

In Tables 1-2, the amount of the components is given in % by weight relative to the total weight of the composition.

### Example 2: Assessment of the remineralization ability of dental anhydrous varnish compositions of the present invention

The aim is to compare the ability of a composition of the invention to repair enamel lesions with that of competitor products which do not comprise any calcium silicate compound.

### Materials and Methods

For this goal, a Knoop hardness test was carried out.

The surface Knoop microhardness has been measured on:
- first, a sound bovine enamel square sample,
- then, the same bovine enamel square sample after introduction of artificial white spot lesions (WSL), and
- finally, the same bovine enamel square sample where white spot lesions (WSL) have been introduced, after being treated with a given composition and soaked in artificial saliva for 7 days in a given composition.

Each measure has been repeated 5 times.

The compositions where bovine enamel square samples with white spot lesions (WSL) have been soaked in are:
- Composition N°1 of the present invention according to Table 1 of Example 1, said composition comprises only one remineralizing agent which is tricalcium silicate;
- compositions N°3-5 of the present invention, which were prepared according to Table 1 of Example 1, said compositions comprise tricalcium silicate as remineralizing agent in combination with at least one other remineralizing agent (sodium fluoride and/or nano hydroxyapatite);
- MI Varnish, commercial composition sold by GC, which does not comprise any calcium silicate compound as remineralizing agent. Indeed, MI Varnish uses 5% of sodium fluoride and 2% of casein phosphopeptides-amorphous calcium phosphate (CCP-ACP) as remineralizing agents. MI Varnish comprises in its composition a resin which is hydrogenated rosin, and
- Duraphat, commercial composition sold by Colgate, which does not comprise any calcium silicate compound as remineralizing agent. Indeed, Duraphat only comprises sodium fluoride as remineralizing agent. Duraphat comprises in its composition a resin which is colophonium.

Based on the measured surface Knoop microhardnesses, the hardness recovery is then calculated to evaluate the remineralizing effect of each composition.

### Results

The hardness recovery values are presented in Table 3.

**Table 3**

| Composition | hardness recovery (%) |
|---|---|
| N°1 of the present invention | 48 |
| N°3 of the present invention | 68 |
| N°4 of the present invention | 71 |
| N°5 of the present invention | 58 |
| MI Varnish | 14 |
| Duraphat | 16 |

The results thus obtained show that the hardness recovery values of compositions according to the present invention are higher compared to that of MI Varnish or Duraphat. Indeed, compositions N°1, 3-5 of the present invention, which were prepared according to Table 1 of Example 1, display hardness recovery values greater than or equal to 48 % whereas the commercial products MI Varnish or Duraphat display hardness recovery values that do not exceed 16 %. Therefore, compositions comprising at least one calcium silicate compound according to the present invention display higher remineralizing effect on enamel lesions than competitor products.

Moreover, when the composition of the invention further comprises another remineralizing agent in combination with the calcium silicate compound, a synergic effect is observed and the remineralizing properties thus obtained on the enamel are even better Example 3: Assessment of the occlusion properties of dental anhydrous varnish compositions of the present invention

The aim is to compare the ability of a composition according to the present invention to occlude tubules with that of a competitor varnish product that does not comprise any calcium silicate compound in its formula. The better the occlusion properties are, the greater the reduction of pain related to hypersensitivity by tubuli blocage is.

### Materials and Methods

For this goal, a test comprising the following steps was carried out:
a) a bovine enamel square sample was submitted to a phosphoric acid treatment,
b) the bovine enamel square sample of step a) was further submitted to a water treatment,
c) a layer of varnish composition was applied on the surface of the bovine enamel square sample of step b),
d) the bovine enamel square sample of step c) was soaked for 7 days in artificial saliva,
e) a Scanning Electron Microscopy (SEM) analysis of the surface of the bovine enamel square sample of step d) is performed, and then
f) based on the SEM image obtained in step e), the percentage of tubules occluded is calculated.

The varnish compositions applied on the surface of the bovine enamel square sample of step b) are:
- compositions N°8 and N°12-15 of the present invention, which were prepared according to Table 1 and Table 2 of Example 1 respectively, and
- Duraphat, commercial composition sold by Colgate.

### Results

The percentage values of tubules occluded are presented in Table 4.

**Table 4**

| Composition | tubules occluded (%) |
|---|---|
| N°8 of the present invention | 35 |
| N°12 of the present invention | 62 |
| N°13 of the present invention | 73 |
| N°14 of the present invention | 50 |
| N°15 of the present invention | 71 |
| Duraphat | 30 |

The results thus obtained show that the percentage values of tubules occluded of compositions according to the present invention are higher compared to that of Duraphat. Indeed, when tricalcium silicate is used alone as remineralizing agent (see composition N°8), the occlusion of tubules are slightly better as Duraphat. When tricalcium silicate compound is used in combination with other remineralizing agents such as in compositions N°8 and N°12-15 of the present invention, the percentage values of tubules occluded ranges from 50 % to 73%, whereas the commercial composition Duraphat displays a percentage value of tubules occluded of only 30%. Therefore, compositions according to the present invention display higher occlusion properties than competitor products which do not comprise calcium silicate compounds in their formulas.

### Example 4: Assessment of the stability of a dental anhydrous varnish composition of the present invention

### Materials and Methods

An ICH test was carried out on the composition N°1 of the present invention, which was prepared according to Table 1 of Example 1.

The composition was stored with or without an headspace in several conditions:
- at 25 °C with 60 % relative humidity (RH) for a short period (1 month), an intermediate period (3 months) and a long-term period (6 months), and
- at 40 °C with 75 % relative humidity (RH) for a short period (1 month), an intermediate period (3 months) and a long-term period (6 months).

### Results

The stability results of the composition N°1 of the present invention are presented in Table 5.

**Table 5**

| | 1 month | 3 months | 6 months |
|---|---|---|---|
| 25 °C, 60 % RH, with headspace | Unchanged | - | - |
| 25 °C, 60 % RH, without headspace | Unchanged | Unchanged | Unchanged |
| 40 °C, 75 % RH, with headspace | Unchanged | - | - |
| 40 °C, 75 % RH, without headspace | Unchanged | Unchanged | Unchanged |

The results thus obtained show that the composition N°1 of the present invention displays great stability over time in several conditions.

## Claims

1. A dental anhydrous varnish composition comprising:
- at least one calcium silicate compound, preferably in an amount ranging from 0.1 % to 20 % by weight relative to the total weight of the composition;
- at least one film-forming agent; and
- at least one volatile organic solvent.

2. The composition according to claim 1, wherein the at least one calcium silicate compound is chosen from wollastonite, dicalcium silicate C2S, tricalcium silicate C3S and mixtures thereof; preferably from dicalcium silicate C2S, tricalcium silicate C3S and a mixture thereof; more preferably the at least one calcium silicate compound is dicalcium silicate C2S or tricalcium silicate C3S; and even more preferably the at least one calcium silicate compound is tricalcium silicate C3S.

3. The composition according to claim **1** or claim **2**, wherein the amount of the at least one calcium silicate compound ranges from 0.1 % to 15 % by weight, preferably from 0.5 % to 15 % by weight, more preferably from 1% to 10% by weight, even more preferably from 1% to 8% by weight, and better still from 2.5% to 7.5% by weight relative to the total weight of the composition.

4. The composition according to any one of claims **1** to **3**, wherein the at least one film-forming agent is selected from resins and/or film-forming polymers; said film-forming polymers preferably being selected from polymethacrylates such as polymethylmethacrylates, polyvinylpyrrolidones, polysaccharides such as cellulose, and mixtures or copolymers thereof; more preferably the at least one film-forming polymer is a polysaccharide selected from cellulose ethers, cellulose esters, and mixtures or copolymers thereof; even more preferably the at least one film-forming polymer is a cellulose ether.

5. The composition according to any one of claims **1** to **4**, wherein the amount of the at least one film-forming agent ranges from 1 % to 40 % by weight, preferably from 5 % to 35 % by weight, more preferably from 8 % to 30 % by weight and better still from 10% to 25% by weight relative to the total weight of the composition.

6. The composition according to any one of claims **1** to **5**, wherein the at least one volatile organic solvent is chosen from volatile alcohols, volatile esters and mixtures thereof; preferably from ethanol, ethyl acetate, isoamyl propionate and mixtures thereof; more preferably from ethanol, ethyl acetate and a mixture thereof; and even more preferably the at least one volatile organic solvent is ethanol.

7. The composition according to any one of claims **1** to **6**, wherein the amount of the at least one volatile organic solvent is greater than or equal to 20 % by weight, preferably ranges from 20 % to 95 % by weight, more preferably from 40 % to 90 % by weight, and better still from 60 % to 80% by weight relative to the total weight of the composition.

8. The composition according to any one of claims **1** to **7**, wherein it further comprises at least one remineralizing agent, different from the at least one calcium silicate compound, preferably chosen from fluoride salts, calcium carbonate, calcium sulfate, hydroxyapatite, a bioglass and mixtures thereof; more preferably chosen from sodium fluoride, tin fluoride, potassium fluoride, cesium fluoride, silver fluoride, calcium carbonate, calcium sulfate, nano hydroxyapatite, a bioglass and mixtures thereof; even more preferably from sodium fluoride, nano hydroxyapatite, a bioglass and mixtures thereof; and better still from nano hydroxyapatite, a bioglass and a mixture thereof.

9. The composition according to claim **8**, wherein the amount of the at least one remineralizing agent, different from the at least one calcium silicate compound, ranges from 0.1 % to 20 % by weight, preferably from 1 % to 19 % by weight, more preferably from 2 % to 18 % by weight, and even more preferably from 2.5 % to 15 % by weight relative to the total weight of the composition.

10. The composition according to claim **8** or claim **9**, wherein it further comprises at least two distinct remineralizing agents, different from the at least one calcium silicate compound and different from one another.

11. The composition according to any one of claims **1** to **10**, wherein it further comprises at least one stabilizer, different from the at least one film-forming agent, preferably chosen from glycerol, sorbitol, polyethylene glycols, propylene glycol, microcrystalline cellulose, silica and mixtures thereof; more preferably from polyethylene glycols, propylene glycol and a mixture thereof; and even more preferably the at least one stabilizer is a polyethylene glycol, preferably a polyethylene glycol having a molar mass of lower than or equal to 600 g/mol, and more preferably of lower than or equal to 350 g/mol; the amount of the at least one stabilizer preferably ranges from 0.5 % to 10 % by weight, more preferably from 1 % to 8 % by weight, and even more preferably from 1.5 % to 5 % by weight relative to the total weight of the composition.

12. Composition according to claims **1** to **11** for use in the treatment and/or the prevention of hypomineralization related pathologies and/or acid attacks.

13. Composition for use according to claim **12**, wherein said composition is applied on at least one surface of at least one tooth of a patient.

14. A dental product comprising at least one compartment package containing the dental anhydrous varnish composition according to any one of claims **1** to **13**.

15. A dental adhesive film directly obtained by a method comprising:
a) applying the dental anhydrous varnish composition according to any one of claims **1** to **13** on at least one surface of at least one tooth of a patient in need thereof; and
b) optionally, drying the composition applied on the surface in step a) to obtain the dental adhesive film on said surface.
